# EUROPEAN PATENT APPLICATION

(11) **EP 1 559 791 A2**
(43) Date of publication of application: **03.08.2005**
(21) Application number: 04016483.2
(22) Date of filing: 13.07.2004
(51) Int. Cl.: C12Q 1/68

(54) **Detection and quantification of siRNA on micro-arrays**

(30) Priority: 11.08.2003 US 637656
(71) Applicant: Eppendorf Array Technologies SA, B-5000 Namur (BE)
(72) Inventor: Remacle, José, 5020 Malonne (BE); Hamels, Sandrine, 1474 Ways (BE); de Longueville, Francoise, 5360 Natoye (BE)
(74) Representative: Becker Kurig Straus

(57) **Abstract**

The present invention relates to a new method for the detection, identification and/or quantification of multiple gene-specific siRNA or stRNA, respectively, the inducers of RNAi. In particular the present invention relates to a method for detecting the presence or change in concentration of siRNA in a cell, which change may be induced by environmental conditions.

### Annex to the application documents-subsequently filed sequences listing

SEQUENCE LISTING

<110> Eppendorf Array Technologies, S.A.

<120> Detection and quantification of siRNA on micro-arrays

<130> 80437 EP

<140> EP 04016483.2
<141> 2004-07-13

<160> 4

<170> PatentIn version 3.1

<210> 1
<211> 18
<212> DNA
<213> artificial sequence

<220>
<223> example
<400> 1
uuuaaccgca tccttctc 18

<210> 2
<211> 20
<212> DNA
<213> artificial sequence

<220>
<223> example
<400> 2
tactaatacg actcactaaa 20

<210> 3

<211>  30

<212>  DNA

<213>  artificial sequence


<220>

<223>  example

<400>  3
gactagctgg aattcaagga tgcggttaaa                                        30

<210>  4

<211>  30

<212>  DNA

<213>  artificial sequence


<220>

<223>  example

<400>  4
cagccaacgg aattcatacg actcactaaa                                        30

## Description

### Field of the invention

The present invention relates to a new method for the detection, identification and/or quantification of multiple gene-specific siRNA or stRNA, respectively, the inducers of RNAi. In particular the present invention relates to a method for detecting the presence or change in concentration of siRNA in a cell, which change may be induced by environmental conditions.

### Description of the related art

In experiments, during which dsRNA was injected into the nematode *Caenorhabditis elegans* it was found that a silencing of genes highly homologous in sequence to the delivered dsRNA occurred (Fire et al., Nature **391** (1998), 806-811). Based on this finding the term "RNA interference" (RNAi) was created nominating the capability of such dsRNA-molecules to affect the translation of transcripts.

During ensuing research in this area it has been shown that dsRNA triggers degradation of homologous RNAs within the region of identity with the dsRNA (Zamore et al., Cell 101 (2000), 25-33). Apparently, the dsRNA is processed to RNA fragments exhibiting a length of about 21-23-ribonucleotides (Zamore et al., supra). These short fragments were also detected in extracts prepared from *Drosophila melanogaster* Schneider 2 cells that were transfected with dsRNA before cell lysis (Hammond et al., Nature **404** (2000), 293-296) or after injection of radiolabelled dsRNA into *D. melanogaster* embryos (Yang et al., Curr. Biol. **10** (2000), 1191-1200) or *C. elegans* adults (Parrish et al., Mol. Cell **6** (2000), 1077-1087).

RNAi was observed to also be naturally present in a wide range of living cells. E.g. these kind of molecules has been found to exist in insects (Kennerdell and Carthew, Cell **95** (1998), 1017-1026), frog (Oelgeschlager et al., Nature **405** (2000), 757-763), and other animals including mice (Svoboda et al., Development **127** (2000), 4147-4156; Wianny and Zemicka-Goetz, Nat. Cell Biol. **2** (2000), 70-75) and also in humans. RNA molecules of similar size have also been found to accumulate in plant tissue that exhibits post-transcriptional gene-silencing (PTGS) (Hamilton and Baulcombe, Sciences **286** (1999), 950-952).

RNAi is closely linked to the post-transcriptional gene-silencing (PTGS) mechanism of co-suppression in plants and quelling in fungi (Cogoni and Macino, Curr. Opin. Microbiol. **2** (1999), 657-662; Catalanotto et al., Nature **404** (2000), 245; Dalmay et al., Cell 101 (2000), 543-553; Ketting and Plasterk, Nature **404** (2000), 296-298; Mourrain et al., Cell 101 (2000), 533-542; Smardon et al., Curr. Biol. **10** (2000), 169-178), and some components of the RNAi machinery are also necessary for post-transcriptional silencing by co-suppression (Catalanotto et al., Nature **404** (2000), 245; Demburg et al., Genes & Dev. **14** (2000), 1578-1583; Ketting and Plasterk, Nature **404** (2000), 296-298).

The natural function of RNAi and co-suppression appears to be protection of the genome against invasion by mobile genetic elements, such as transposons and viruses, which produce aberrant RNA or dsRNA in the host cell when they become active (Jensen et al., Nat. Genet. **21** (1999), 209-212; Ketting et al., Cell **99** (1999), 133-141; Ratcliff et al., Plant Cell **11** (1999), 1207-1216; Tabara et al., Cell **99** (1999), 123-132; Malinsky et al., Genetics **156** (2000), 1147-1155). Specific mRNA degradation prevents transposon and virus replication, although some viruses seem to be able to overcome or prevent this process by expressing proteins that suppress PTGS (Anandalakshmi et al., Science **290** (2000), 142-144; Lucy et al., EMBO J. **19** (2000), 1672-1680; Voinnet et al., Cell **103** (2000), 153-167).

The currently existing model for the mechanism of RNAi is based on the observation that the introduced dsRNA is bound and cleaved by RNase III-like enzyme Dicer to generate products having the length indicated above. These molecules, termed small interfering RNAs (siRNAs) trigger the formation of RNA-induced silencing complex (RISC). The resulting dsRNA-protein complexes appear to represent the active effectors of selective degradation of homologous mRNA (Hamilton and Baulcombe, Sciences **286** (1999), 950-952, Zamore et al., Cell **101** (2000), 25-33; Elbashir et al., Genes & Dev. **15** (2001), 188-200. Elbashir et al. provide evidence that the direction of dsRNA processing determines, whether sense or antisense target RNA can be cleaved by the siRNA-protein complex. Helicases in the complex unwind the dsRNA, and the resulting single-stranded RNA (ssRNA) seems to be used as a guide for substrate selection. Once the ssRNA is base-paired with the target mRNA, a nuclease activity, presumably within the complex, degrades the mRNA.

The enzyme which produces the siRNA also produces other type of small RNA molecules termed microRNA (miRNA) Theses miRNA are processed from endogenous transcripts that from hairpin structures. The miRNA formed are involved into the control of other genes by binding to the 3' end of their messenger RNA in animals (Chi et al, Proc. Natl. Acad. Sci. **100** (2003), 6343-6346).

Thus, RNAi seems to be an evolutionary conserved mechanism in both plant and animal cells that directs the degradation of mRNA homologous to siRNA. The ability of siRNA to direct gene silencing in mammalian cells has raised the possibility that siRNA might be used to investigate gene function in a high throughput fashion or to specifically modulate gene expression in human diseases.

In US-P-6,506,559 a process of introducing dsRNA into a living cell is described, so as to inhibit gene expression of a target gene in that cell. Inhibition is sequence specific in that the nucleotide sequences of the duplex region of the RNA and of a portion of the target gene are identical.

In WO-02/44321 chemically synthesized siRNA duplexes of 19-25 nucleotides with overhanging 3'-ends are provided to mediate efficient target mRNA cleavage.

In WO-03/006477 another method of inducing gene silencing is disclosed. Engineered dsRNA precursors are provided, which upon expression in a cell are processed by the cell to produce siRNAs that selectively silence targeted genes using the cell's own RNAi pathway.

US-A-20020173478 and 20030084471 show the applicability of RNAi to mammalian cells including human cells and cell lines, and propose these molecules for administration to human patients.

Other methods have been described for modulating RNAi pathway activity. In WO-01/29058 genes are provided, the expression products of which being involved in the mediation of RNAi. RNAi requires a set of conserved cellular factors to suppress gene expression. These factors are the components of the RNAi pathway (e.g. RDE-1, RDE-4) and provide activities necessary for interference. These activities may be absent or not sufficiently activated in many cell types to induce RNAi.

In most of the experiments, siRNA has been introduced into the cells in order to inhibit transcription of the genes but without knowledge of the naturally occurring siRNA.

The detection of naturally occurring siRNA is difficult to perform given their number, their small size and their low number in the cells. One method was proposed based on the cloning of siRNAs after addition of linker segments to their 5'- and 3'-termini using T4 ligase and amplification of the elongated RNA (Elbashir et al., Gene & Dev. 15 (2001), 188-200). The analysis of the cloned fragments was performed by sequencing. This cloning method allows the analysis of siRNA one by one only and is, therefore, time consuming and expensive. None of the previous documents provide an easy method for the multiple analysis of the naturally occurring siRNA, the inducer of RNAi.

Thus, there is a need for an improved method for the detection and identification of RNAi naturally present in the cells.

An object of the present invention, therefore, resides in providing a method for rapidly and reliably detecting and identifying RNAi, that is naturally present in a cell.

### Description of the invention

The present invention provides a method based on the use of micro-arrays for the specific detection of the siRNA molecules directed to specific genes and being present in cells or cell extracts. In particular, the present method is designed for the detection, identification and/or quantification of a siRNA directed against at least one specific gene present in a sample and comprises the steps of, (i) providing an array onto which a number of polynucleotides corresponding to at least one gene and or transcript of a cell are arranged on pre-determined locations; (ii) isolating siRNA from a target cell; (iii) contacting the siRNA with the array under conditions allowing hybridization of the siRNA to the capture molecules present on the array; (iv) detecting a signal or a change in a signal present on a specific location on the array; wherein the location of the signal on the array and/or change of the signal is indicative of the presence of a siRNA in the cell.

### Description of the drawings

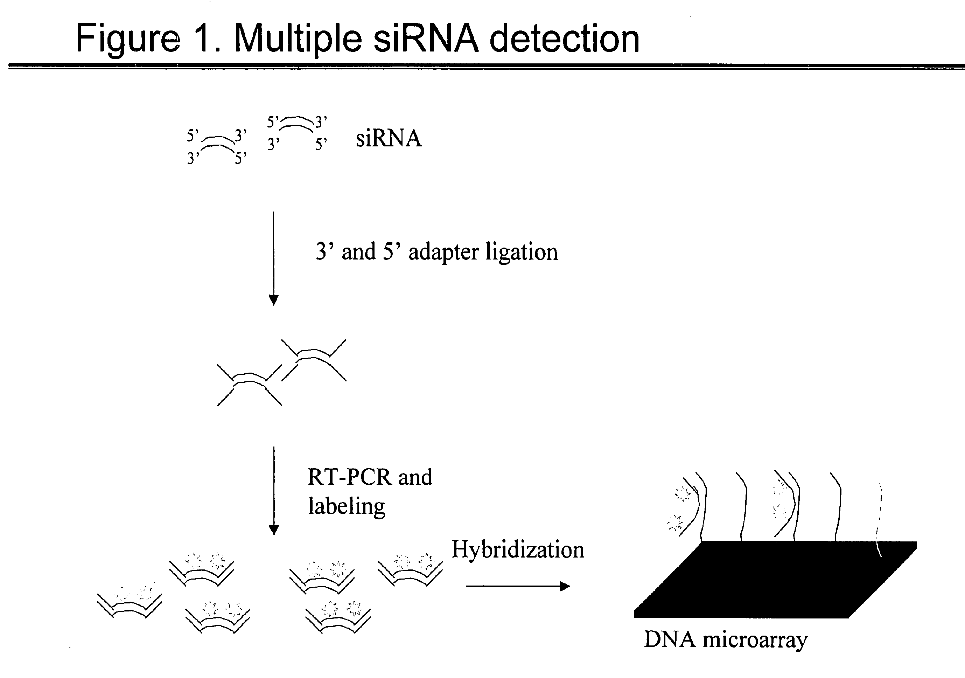

Figure 1 presents an embodiment for the detection of siRNA on arrays. The siRNAs are ligated to adaptors which are then used for binding of primers and amplification by RT-PCR. The amplified labeled amplicons are then detected on a micro-array bearing sequences at least partly identical to the genes for which analysis is required.

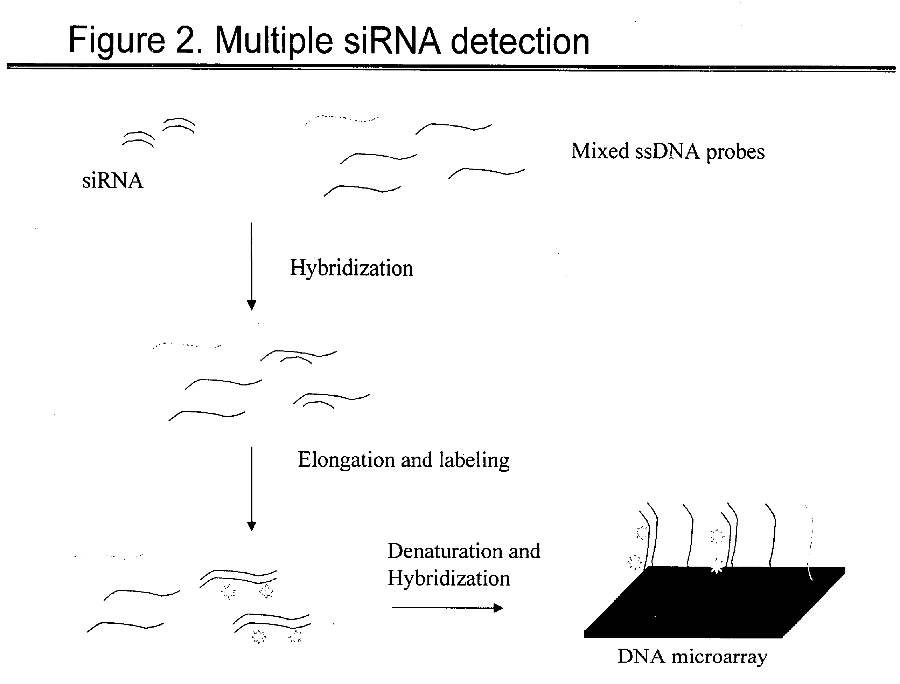

Figure 2 shows another embodiment for the detection of siRNAs in which they are first incubated in solution with their complementary DNA strands and after elongation and labeling, are detected by hybridization on array bearing sequences at least partly identical to the genes for which the analysis is required.

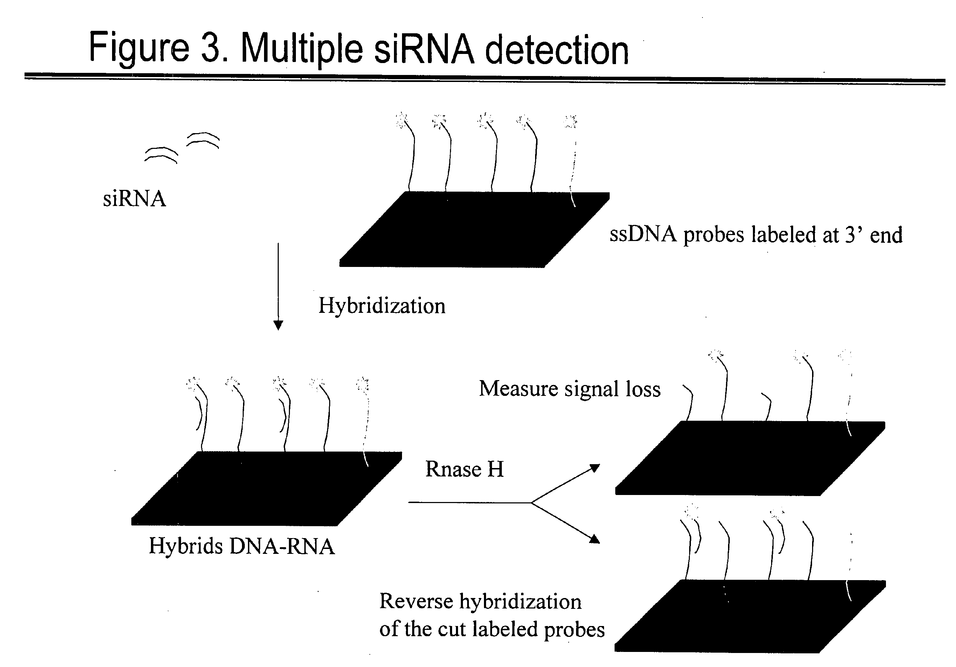

Figure 3 presents another embodiment of the siRNAs detection in which the denatured siRNAs are incubated onto the array having fixed labeled DNA sequences at least partly identical to the genes for which the analysis is required and determining fragments released after treatment with the Rnase H.

### Definitions

The term "genes" shall designate the genomic DNA which is transcribed into mRNA and then translated into a peptides or proteins. The measurement of the expressed genes is performed on either molecules within this process most currently the detection of the mRNA or of the peptide or protein. The detection can also be based on specific property of the protein being for example its enzymatic activity.

The terms "nucleic acid, array, probe, target nucleic acid, bind substantially, hybridizing specifically to, background, quantifying" are as described in the international patent application WO97/27317, which is incorporated herein by reference.

The term "nucleotide triphosphate" refers to nucleotides present in either as DNA or RNA and thus includes nucleotides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleotides present in nucleic acids (either DNA or RNA) compared with the bases of said nucleic acid, and includes nucleotides comprising usual or modified bases as above described.

References to nucleotide(s), oligonucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

The terms "nucleotide species" is a composition of related nucleotides for the detection of a given sequence by base pairing hybridization; nucleotides are synthesized either chemically or enzymatically but the synthesis is not always perfect and the main sequence is contaminated by other related sequences like shorter one or sequences differing by a one or a few nucleotides. The essential characteristic of one nucleotides species for the invention being that the overall species can be used for capture of a given sequence.

"Polynucleotide" sequences that are complementary to one or more of the siRNA described herein, refers to polynucleotides that are capable of hybridizing under stringent conditions to at least part of the nucleotide sequence of said RNA or RNA copies. Given the small size of the siRNA, the capture molecules have to be identical or at least have more than 90% identical sequence in order to specifically detect the siRNA beside other possible flanking regions.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The terms "capture probe" designates a molecule which is able to specifically bind to a given polynucleotide. Polynucleotide binding is obtained through base pairing between two polynucleotides, one being the immobilized capture probe and the other one the target to be detected.

The present invention is based on the use of arrays having multiple single nucleotide sequences arranged on specific, pre-determined locations thereon and being identical or complementary to gene sequences, or mRNAs corresponding to genes, present in the cells for which the siRNA are to be determined.

The main characteristic of the invention is to obtain a direct analysis and an overview of the genes which are essentially affected by the regulation through siRNAs naturally present in the cells. The signals of the different spots related to each gene being a direct measurement of the number and the concentration of the siRNA in the analysed cells or tissues. Also, the invention is not limited by the number of genes to be screened. The array allows to analyse either from 2 to 100 and more preferably until 1000 and still up the entire gene pool present in a cell. This number depends on the species and the differentiation of the cell but can be as large as around 40.000 for the human genome.

### Detailed description of the invention

The present invention provides a method for the detection and/or identification and/or quantification of multiple siRNAs directed against at least one gene or mRNA or their complementary sequence, respectively, and present in a sample by detecting a signal or change in a signal present at a specific location on an array, said signal or signal change at such location being related to the presence of siRNA directed against a particular mRNA.

In principle, the method comprises the steps of providing a support containing an array onto which a number of genes and/or transcripts of a cell is arranged on pre-determined locations.

The support is generically composed of a solid surface which may be selected from the group consisting of glasses, electronic devices, silicon supports, silica, metal or mixtures thereof prepared in format selected from the group of slides, discs, gel layers and/or beads. Beads are considered as arrays in the context of the present invention, as long as they have characteristics which allow a differentiation from each other, so that identification of the beads is correlated with the identification of a given capture probe and so of the target sequence.

On the support, a number of capture molecules is fixed by covalent binding, each capture molecule being located at a specific location and having at least in part a sequence in a single strand form identical to a gene for which the presence of a siRNA is screened.

In principle, the detection of the siRNA may be performed on the (+) or (-) strand of the corresponding gene sequence, since the siRNA contain both of the strands. However, in most applications, use of complementary sequences might lead to a binding of the natural mRNA as well, which might interfere with the analysis. This is the case for the detection method presented in figure 3. In this case the sequence identical to the mRNA has to be present on the surface of the array. In the method proposed in figure 1, the two strands can be used.

Generally, the capture probes may be synthesized by a variety of different techniques, but are preferably synthesized by PCR amplification from cloned genes using an aminated primer. The amino group of the amplicon is then reacted with a functionalized surface bearing reactive groups, such as, but not limited, to aldehyde, epoxide, acrylate, thiocyanate, N-hydroxysuccinimide. After having formed a covalent linkage, the second strand of the amplicon is then removed by heating or by alkaline treatment so that single strand DNA or RNA is present on the surface and ready to bind to the complementary siRNA or siRNA copies.

Given the progress of chemical synthesis of the nucleotides, the use of chemically synthesised nucleotides is also envisaged in the invention. The synthesised nucleotides are also preferably aminated or thiolated and deposited on the functionalized surface. Advantage of the chemically synthesised nucleotides is their easy production.

In a preferred embodiment the array contains capture probes represented by polynucleotides having a sequence identical to essentially the full length mRNA. In another embodiment, the array contains capture probes being polynucleotides having a sequence complementary to essentially the full length mRNA.

According to another embodiment, the array contains a number of capture probes being polynucleotides identical or complementary to at least different parts of the same mRNA sequence, which capture probes are present at specific (and different) locations of the array. Preferably, the different capture molecules present on the array cover most and preferably all of the gene sequences or mRNA present in a cell. The amount of siRNA for one gene may then be calculated as the sum of the signals for the different parts of the same gene.

In a preferred embodiment, the capture molecules are present at a density superior to 10 fmoles, and preferably 100 fmoles per cm² surface of the solid support. In another embodiment capture probes are present on different supports being preferentially beads with chemical or physical characteristics for their identification with a specific capture probe. The simplest array would contain one capture probe.

The invention also embraces the support and its substrate on which is bound the capture molecules for the detection of given siRNA target molecules.

As the genes, any known gene sequence derived from the genome of an organism may be used. Alternatively, parts of the said gene are put to use, which are preferably derived from the coding regions, i.e. the exons. According to a preferred embodiment transcripts of the genes are used and arranged on the array, i.e. nucleotides derived from the mRNA-pool of the cell to be investigated. Methods of arranging nucleotides and polynucleotides are well known in the art and may be found in Bowtell, D. and Sambrook ( DNA Microarrays, J. Cold Spring Harbor Laboratory Press, 2003 Cold Spring Harbor, New York, pg 1-712) which document is incorporated herein by way of reference. In a preferred embodiment the nucleotide sequence is attached to the support via a linker, which may be a polynucleotide exhibiting a length of between about 20 to 200 nucleotides ( EP 1 266 034 ). In principle, the capture probes may be DNA, PNA or RNA.

In a next step (step (ii)), siRNA from a cell of interest is isolated. An exemplary process for the isolation of siRNA is described e.g. in Tuschl et al. 1999, which document is incorporated herein by way of reference.

The siRNA once isolated may directly be used for the assay or, preferably, may be processed further prior to performing the assay.

According to a preferred embodiment, the siRNA will be labelled prior to its use. The labelling may be performed by attaching a specific molecule to the siRNA, that may be detected, e.g. via fluorescence, colorimetry, chemo- or bioluminescence, electric, magnetic or particularly biotin. Biotin-labelled nucleotides may be attached/incorporated, which is then recognized by binding proteins being either antibodies or streptavidin or related binding molecules. The binding proteins are labelled by any chemical or physical means and detected and quantified. Indirect labelling is also of use when amplification of the signal is required.

A labelling of the siRNA may also be performed by incubating the siRNA with a mixture of ssDNA probes under conditions as to obtain formation of a RNA-DNA hybrid complex, whereupon an elongation and concurrent labelling of the small RNA may be achieved. Here, the ssDNA is used as a matrix and labelled ribonucleotide/deoxynucleotides are utilized for the elongation. The ssDNA used for the formation of the hybrid complex can be replaced by any nucleotide or nucleotide-like molecules as long as the elongation of the bound siRNA is possible. After denaturation the labelled strand will be used for incubation with the capture probes present on the array for detection and/or quantification of the transcripts.

In an alternative embodiment, the capture probes present on the array may contain a label at their 3'-end. After binding of the siRNA, the RNA/DNA hybrids are then cleaved with Rnase H thus releasing the label from those capture probes, where the siRNA had bound. Therefore, in this embodiment, the decrease in signal is representative of the presence of a siRNA present in the sample.

The sequence of the siRNA may also be determined by performing additional analysis of the release sequence.

In an first embodiment, the released fragments may be detected and/or quantified and/or identified by their hybridization on specific capture probes present on a second DNA microarray (cf. Fig. 3). In a second embodiment, the released fragments are separated, identified and/or quantified after electrophoresis. The size of the released fragments indicate the location of binding of the siRNA and allow their identification. Also a sequence analysis of released sequence will lead to the same identification (figure 3).

The siRNA may also be transcribed to their corresponding DNA-copies or amplified by means of PCR. Accordingly, the copying may be performed using a retro-transcriptase allowing for the incorporation of labelled nucleotides in the forming strand. Also, the siRNA may be subjected to a PCR-reaction, which in principle involves the use of 3'- and 5'-adapter oligonucleotides in order to perform a blunt end ligation with the multiple extracted siRNAs in solution. The product thus obtained is then reverse transcribed with a 3'-RT primer complementary to the 3'-adapter. Subsequently, a PCR amplification cycle is then perform with a 5'-primer complementary of the cDNA and in the presence of the 3'-RT primer. Labeled nucleotides are incorporated into the amplicons during the PCR-reaction.

In a next step (step (iii)), the siRNA or molecule derived therefrom (e.g. a DNA-copy or amplicon), is contacted with the array under conditions, allowing hybridization of the siRNA, or the molecule derived therefrom, with the capture probes present on the array. After a time sufficient for forming the duplex, a signal or a change in signal is detected on a specific location on the array.

In case the siRNA, or molecule derived therefrom, has been labelled prior to the hybridization step, the presence of fixed labelled target will be indicative of the presence of siRNA in the sample and, in knowledge of the gene to which it binds, also which transcript is controlled in the cell via this mechanism. The amount of fixed labelled target on the array will be proportional to the siRNA if performed under the appropriate conditions.

The presence of target bound on the different capture probes present on the solid support may be analyzed, identified and/or quantified by an apparatus comprising a detection and/or quantification device of a signal formed at the location of the binding between the target molecule and the capture molecule, preferably also a reading device of information recorded on a surface of said solid support, a computer program for recognizing the discrete regions bearing the bound target molecules upon its corresponding capture molecules and their locations, preferably also a quantification program of the signal present at the locations and a program for correlating the presence of the signal at these locations with the diagnostic and/or the quantification of thecomponents to be detected according to the invention.

The principle laid down in the present specification may also be used in a method for determining the exact location of the siRNA binding on a gene sequence and/or the transcript. To this end, sequences of the gene or transcript, respectively, are arranged on the array on different locations, and upon hybridization it may be determined, to which part of the gene and/or transcript the siRNA binds.

The knowledge provided by the present invention allows the design of new medicaments comprising sequences containing the RNAi sequences.

Also, the present invention is suitable for screening for compounds appropriate for regulation of gene translation or to follow cell reactions in the presence of biological or chemical compounds.

According to one embodiment, the cells, tissues or organisms are placed in the presence of one molecule and the analysis according to the present invention is carried out. The analysis of the spots intensities specific of the different genes gives an estimation and possible quantification of the siRNA present within the cells compared to cells incubated without the given compound. The invention is particularly useful for the determination of the efficiency of the transfection of the siRNA directed against one or several particular genes.

Variation in the level of the siRNA for particular genes are determined and give a first overview of the changes occurring in the biological organisms, cells or tissues, due to the compound. Compounds comprise: biological molecules such as cytokines, growth hormones, or any biological molecules affecting cells. Is also comprises chemical compounds such as drugs, toxic molecules, compounds from plants or animal extracts, chemicals resulting from organic synthesis including combinatory chemistry. The invention is particularly well suited for the screening of these compounds on cell regulation of the transcription of the genes. The overview of the changes in biological organisms is best obtained by screening for potentially active siRNA directed against the main vital cellular functions as following: apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes . The invention best application is for the detection of si RNA against genes corresponding for the proteins involved in at least 9 of the 13 main cellular functions. Also on particular embodiment, the array is used for the identification and/or quantification of siRNA present in cells against gene corresponding to at least 5 genes from one cellular functions including the 13 vital function here above described but also including specialized functions such as cell differentiation, oncogene/tumor suppressor, stress response, lipid metabolism, proteasome, circulation . Also the invention is best when focussed on genes related to one particular function which has biological, pharmaceutical, therapeutical or pathological interest.

In one embodiment, cells, tissues or organisms are incubated in particular physical, chemical or biological conditions and the analysis performed according to the invention. The particular physical conditions means only conditions in which a physical parameter has been changed such as pH, temperature, pressure.

The particular chemical conditions mean any conditions in which the concentrations of one or several chemicals have been changed as compared to a control or reference condition including salts, oxygen, nutriments, proteins, glucides (carbohydrates), and lipids.

The particular biological conditions mean any changes in the living cells, tissues or organisms including ageing, stress, transformation (cancer), pathology, which affect cells, tissues or organisms.

Therefore, the method and support as described herein may be utilized as part of a diagnostic and/or quantification kit which comprises means and media for analyzing biological samples containing target molecules being detected after their binding onto the capture probes being present on the support in the form of array with a density of at least 4 different capture probes per cm² of surface of rigid support. In its simple specification, the kit may also contain a support with a single capture probe.

Also provided by the present invention is a kit for the detection and/or identification and/or quantification of multiple siRNA directed against at least one gene present in a sample comprising, which kit comprises an array, harboring capture probes having a sequence identical or complementary to a mRNA or parts thereof and being present at specific locations of the array, and buffers and labels.

Also provided is a screening device for testing the effects of a compound on gene expression by detection of the presence of siRNA directed against at least one gene, said screening device comprising an array including capture probes having a sequence identical or complementary to mRNA or part thereof and being present at specific locations of the array and optionally buffers and labels.

The following example illustrate the invention without limiting it thereto.

### Example

siRNAs were extracted from the cell medium and subsequently treated with proteinase K and were separated on a denaturing 15% polyacrylamide gel. A band, including a size range of at least 18-24 nt, was excised and then eluted into 0.3 M NaCl overnight at 4°C in siliconized tubes. The RNA was recovered by ethanol precipitation and then dephosphorylated (30 µl reaction, 50 °C, 30 min, 10 U alkaline phosphatase; Roche).

The reaction was stopped by phenol/chloroform extraction, and the RNA was ethanol precipitated. The 3' adapter oligonucleotide (pUUUaaccg catccttctcx: uppercase, RNA; lowercase, DNA; p, phosphate; x, 4-hydroxymethylbenzyl) was then ligated to the dephosphorylated ∼21-nt RNA (20 µL reaction, 37°C, 30 min, 5 µM 3' adapter, 50 mM Tris-HCl at pH 7.6, 10 mM MgCl2, 0.2 mM ATP, 0.1 mg/ml acetylated BSA, 15% DMSO, 25 U T4 RNA ligase; Amersham-Pharmacia) (Pan and Uhlenbeck 1992). The ligation reaction was stopped by the addition of an equal volume of 8 M urea/50 mM EDTA stopmix and directly loaded on a 15% gel. The ligation product was recovered from the gel and 5' phosphorylated (20 µL reaction, 37°C, 30 min, 2 mM ATP, 5 U T4 polynucleotide kinase; NEB).

The phosphorylation reaction was stopped by phenol/chloroform extraction, and RNA was recovered by ethanol precipitation. Next, the 5' adapter (tactaatacgactcactAAA: uppercase, RNA; lowercase, DNA) was ligated to the phosphorylated ligation product as described above. The new ligation product was gel purified and eluted from the gel slice in the presence of reverse transcription primer (GACTAGCT**GGAATT**CAAG GATGCGGTTAAA: bold, *Eco*RI site), used as carrier. Reverse transcription (15 µL reaction, 42°C, 30 min, 150 U Superscript II reverse transcriptase; Life Technologies) was followed by PCR using a 5' primer CAGCCAACG**GAATTC**ATACGACTCAC TAAA (bold, *Eco*RI site), the 3' RT primer and biotin-dATP/biotin-dCTP mix (10 µM each).

The labelled PCR product was then hybridized on the Dual Chips Human General micro-array bearing ssDNA capture probes specific for 202 genes (Eppendorf, Hamburg, Germany). The dual chips Human General contains capture molecule having sequences identical at least in part to 202 genes belonging to the 13 main vital cell functions. The labelling of the bound targets was obtained as described by Delongueville et al (Biochem Pharmacol. 2002, 64:137-49) using Cy3 labelled antibodies against biotin and the arrays scanned using a laser confocal scanner "ScanArray" (Packard, USA) at a resolution of 10 µm.

After image acquisition, the scanned 16-bit images were imported to the software, 'ImaGene4.0' (BioDiscovery, Los Angeles, CA, USA), which was used to quantify the signal intensities. The spots intensities for every genes were first corrected by a subtraction of the local background intensity from signal intensity.

In order to evaluate the entire experiment, several positive and negative controls (for hybridization and detection) are first analysed. Then the signal obtained on each siRNA spots is analysed in order to correlate the result with the presence or not of siRNA directed against the specific gene in the sample.

## Claims

1. A method for the detection and/or identification and/or quantification of a siRNA directed against at least one specific gene present in a sample comprising the steps of:
(i) providing an array onto which are fixed polynucleotides corresponding to at least one gene and/or transcript of a cell, said polynucleotides being arranged on pre-determined locations;
(ii) isolating siRNA from a target cell;
(iii) contacting the siRNA with the array under conditions allowing hybridization of the siRNA to the capture molecules present on the array;
(iv) detecting a signal or a change in a signal present on a specific location on the array;
wherein the location of the signal on the array and/or change of the signal is indicative of the presence of a siRNA in the cell.

2. The method according to claim 1,
wherein before step (iii) the siRNA is labelled and/or enzymatically copied and optionally amplified, and/or
wherein the detection of the siRNA is performed after elongation of the siRNA on one of its complementary sequence, and/or
wherein after step (iii) the RNA-DNA hybrids are cut and loss of the capture molecule is determined.

3. The method according to claim 2, wherein the loss of the capture molecule is determined as the decrease of signal at a particular location compared to a control.

4. The method according to claim 2, wherein the fragment of the capture molecule released is determined and optionally identified or sequenced.

5. The method according to the preceding claims, wherein the capture probe is
a polynucleotide having a sequence identical to a mRNA; or
a polynucleotide having a sequence complementary to a mRNA; or
a polynucleotide having at least part of a sequence identical to a mRNA; and/or
a molecule selected from DNA, PNA or RNA.

6. The method according to any of the claims 1 to 5,
wherein the micro-array bear several capture probes having a sequence identical to at least different parts of the same mRNA sequence; or
wherein the micro-array bear several capture probes having a sequence complementary to at least different parts of the same mRNA sequence.

7. The method according to any of the claims 1 to 6 wherein the micro-array bear capture probes for the determination of the siRNA directed against genes corresponding to different proteins involved in at least 9 of the 13 main cellular functions including apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription and house keeping genes.

8. The method according to any of the claims 1 to 6 wherein the micro-array bear capture probes for the determination of the siRNA directed against at least 5 genes corresponding to proteins involved in at least one of the cellular functions including apoptosis, cell adhesion, cell cycle, growth factors and cytokines, cell signaling, chromosomal processing, DNA repair/synthesis, intermediate metabolism, extracellular matrix, cell structure, protein metabolism, oxidative metabolism, transcription, cell differentiation, oncogene/tumor suppressor, stress response, lipid metabolism, proteasome, circulation and house keeping genes.

9. The method according to any of the preceding claims, wherein
the micro-array bear at least 20 different capture probes for the determination of siRNA directed against at least 20 genes, and/or
the micro-array is arranged on several supports having at least one feature particular for the capture molecule in order to be identifiable.

10. The method according to the preceding claims, wherein the detection is effected by fluorescence, colorimetry, chemo- or bioluminescence, electric or magnetic devices.

11. The method according to any of the preceding claims for screening for compounds usable in the regulation of the transcription of the genes.

12. A kit for the detection and/or identification and/or quantification of multiple siRNA directed against at least one gene present in a sample comprising
an array, including capture probes having a sequence identical or complementary to a mRNA or parts thereof and being present at specific locations of the array, and
optionally buffers and labels.

13. A screening device for testing the effect of compounds on the presence of siRNA directed at least against at least one gene said screening device comprising an array including capture probes having a sequence identical or complementary to mRNA or part thereof and being present at specific locations of the array and optionally buffers and labels.
